# EUROPÄISCHE PATENTSCHRIFT

(11) **EP 0 942 684 B1**
(45) Veröffentlichungstag und Bekanntmachung des Hinweises auf die Patenterteilung: **20.09.2000**
(21) Anmeldenummer: 97953679.4
(22) Anmeldetag: 08.11.1997
(51) Int. Cl.: A61B 10/00, A61B 17/28

(54) **MEDIZINISCHE ZANGE**
MEDICAL FORCEPS
PINCE MEDICALE

(30) Priorität: 09.11.1996 DE 19646326
(43) Veröffentlichungstag der Anmeldung: 22.09.1999
(73) Patentinhaber: Lang, Dieter, 96342 Stockheim (DE)
(72) Erfinder: Lang, Dieter, 96342 Stockheim (DE)
(74) Vertreter: Witte, Alexander, Dr.-Ing.
(86) Internationale Anmeldenummer: EP9706215
(87) Internationale Veröffentlichungsnummer: WO9820798

(56) Entgegenhaltungen:
- EP-A- 0 598 607
- US-A- 4 243 047

## Beschreibung

Die Erfindung betrifft eine medizinische Zange zur Gewebeentnahme im menschlichen oder tierischen Körper, mit zwei Maulteilen am patientennahen Ende, die über Griffelemente am patientenfernen Ende relativ zueinander bewegbar sind, wobei die Maulteile Schneiden aufweisen, die beim Schließen der Maulteile schneidend zusammenwirken.

Die Erfindung betrifft ferner eine medizinische Zange zur Gewebeentnahme im menschlichen oder tierischen Körper, mit zwei Maulteilen am patientennahen Ende, die über Griffelemente am patientenfernen Ende relativ zueinander bewegbar sind, wobei die Maulteile beim Schließen zum Fassen von Gewebe stumpf aufeinanderstoßen.

Derartige Zangen zum Abtrennen von Gewebe der zuerst erwähnten Art und derartige Zangen zum Fassen von Geweben der an zweiter Stelle genannten Art sind allgemein bekannt.

Beispielsweise wird eine Zange der zuerst erwähnten Art in US-A-4 243 047 beschrieben.

Zangen zum Abtrennen von Gewebe werden bei einem operativen Eingriff am menschlichen oder tierischen Körper dazu verwendet, Gewebe, beispielsweise Organ- oder Knochengewebe, abzutrennen bzw. herauszuschneiden. Die Zangen weisen dazu an ihrem patientennahen Ende zwei Maulteile auf, die Schneiden, d.h. geschärfte Bereiche mit scharfen Schneidkanten aufweisen, die zum Abtrennen des Gewebes zusammenwirken.

Beispielsweise ist aus dem DE-Prospekt "Karl Storz Endoskope, Endoskopische Chirurgie", Abschnitt 5, Seite SCT 5/4A (Zange nach FRANGENHEIM) eine als durchschneidend bezeichnete Zange bekannt, deren Maulteile Schneidkanten in Längsrichtung der Zange bzw. der Maulteile sowie vordere quer zur Längsrichtung verlaufende Schneidkanten aufweisen. Durchschneidend bedeutet, daß ein bewegliches Maulteil beim Schneiden durch ein ortsfestes hindurch bewegt wird, wobei die Schneiden aneinander vorbei laufen und ein dazwischen erfaßtes Gewebestück abtrennen.

Es sind aber auch Zangen zum Abtrennen von Gewebe bekannt, die nur in Längsrichtung verlaufende Schneiden aufweisen, die also in der Art einer Papierschere in das Gewebe einschneiden.

Ferner sind sogenannte Löffelzangen bekannt, deren beide Maulteile jeweils eine Schneide aufweisen, die beim Schließen der Maulteile aufeinanderstoßen, ohne aneinander vorbeizulaufen, und dabei schneidend zusammenwirken.

All diesen zuvor erwähnten Zangen ist gemeinsam, daß ihre Maulteile als Schneidwerkzeuge ausgebildet sind, d.h. daß die Zangen nur zum Abtrennen von Gewebe verwendbar sind. Mit diesen Zangen ist es nicht möglich, das abgetrennte Gewebe zu fassen und aus dem menschlichen Körper zu entnehmen.

Zur Entnahme der Gewebeproben sind sogenannte Faßzangen vorgesehen, d.h. Zangen der eingangs an zweiter Stelle genannten Art, deren Maulteile nicht als Schneidwerkzeuge ausgebildet sind, sondern als Faßwerkzeuge, die demnach stumpf aneinanderstoßende Flächen aufweisen, zwischen denen das abgetrennte Gewebe gefaßt werden kann, ohne dieses dabei zu zertrennen.

Bei operativen Eingriffen, bei denen Gewebeteile entnommen werden, ist der operierende Arzt daher gezwungen, zum Abtrennen des Gewebes und zum Entnehmen des Gewebes zwei Zangen zu verwenden, nämlich eine Schneidzange und eine Faßzange. Dies hat jedoch den Nachteil, daß sich der Arzt mit der Arbeitsweise von zwei verschiedenen Zangen vertraut machen muß, um für deren Bedienung die erforderliche operative Sicherheit zu bekommen. Da außerdem häufig das zu entnehmende Gewebe nicht bereits mit einem einzigen Schneidvorgang abgetrennt werden kann, muß der Arzt mehrmals die Zange wechseln, um ein zunächst mit der Schneidzange abgetrenntes Gewebestück anschließend mit der Faßzange aus dem Körper zu entfernen. Dies führt dazu, daß der Arzt bei einem Operationsvorgang mehrmals die eine Zange aus der Hand legen und die jeweils andere Zange ergreifen muß.

Ein weiterer Nachteil besteht in dem erhöhten Kostenaufwand derartiger bekannter Zangen, der daraus resultiert, daß zwei komplette Zangen, eine zum Abtrennen und eine zum Fassen, mit Schaft, Betätigungselementen und Griffelementen bereitgestellt werden müssen, obwohl sich die Schneidzange und die Faßzange nur hinsichtlich ihrer Maulteile funktionsbedingt unterscheiden müssen.

Da das Wechseln der Zange von den operierenden Ärzten als nachteilig angesehen wird, versuchen diese mitunter, das durch die Schneidzange abgetrennte Gewebestück mit den Maulteilen der Schneidzange zu fassen, was jedoch die Gefahr beinhaltet, daß beim Abziehen der Zange vom Körper das erfaßte Gewebestück in zwei Teile aufgetrennt wird und diese im Körper verbleiben.

Aus der US 4,711,240 ist ferner ein chirurgisches Instrument bekannt, mit dem Gewebe aus weichen, schwammartigen Organen, wie z.B. aus Nieren oder der Leber, herausgelöst werden kann, ohne dabei die in diesen Organen vorhandenen Blutgefäße zu verletzen. Dazu weist das bekannte Instrument am patientennahen Ende kammartig ausgebildete Trennwerkzeuge auf, die gegeneinander geschlossen werden, wobei die stumpfen Kammzähne ineinandergreifen und das Gewebe von beiden Seiten des Organs aus herauslösen. Die kammartigen Trennwerkzeuge wirken jedoch nicht schneidend zusammen. Das Gewebe des Organs wird beim Schließen der Trennwerkzeuge vielmehr zwischen den Kammzähnen zermalmt und kann dann ausgespült werden. Mit diesem Instrument ist es nicht möglich, das herausgelöste Gewebe zu fassen. An einem der beiden Trennwerkzeuge kann ein Aufsatz aufgesteckt werden, der eine glatte Oberfläche aufweist, und der dann verwendet wird, wenn aus dem Organ nur von einer Seite aus Gewebe herausgelöst werden soll, bspw. zur Entfernung von Nierensteinen.

Ferner ist aus der EP-A-0 598 607 eine chirurgische Zange bekannt, die nicht schneidend ist, sondern zum atraumatischen Fassen von Organen bzw. Blutgefäßen dient, um diese beiseitezubewegen, um das dahinter versteckt liegende Operationsgebiet freizulegen. Dazu sind für die Maulteile atraumatische, lösbar befestigbare Polster vorgesehen, die beim Schließen der Maulteile die erfaßten Organe bzw. Gefäße nicht atraumatisieren oder sogar verletzen. Diese bekannte Zange kann jedoch nicht als Schneidwerkzeug verwendet werden.

Auch bei den zuvor genannten Instrumenten besteht somit der Nachteil, daß sie nur eine Funktion aufweisen, nämlich entweder nur Gewebe herauszutrennen, oder nur Gewebe zu fassen.

Der Erfindung liegt demnach die Aufgabe zugrunde, eine medizinische Zange der eingangs genannten ersten Art und eine medizinische Zange der eingangs an zweiter Stelle genannten Art dahingehend weiterzubilden, daß die Vorgänge des Abtrennens und des Fassens bzw. des Entnehmens des Gewebes nacheinander durchgeführt werden können, ohne daß dazu der Arzt die Zange aus der Hand legen und eine andere Zange ergreifen muß.

Erfindungsgemäß wird die Aufgabe hinsichtlich der eingangs zuerst genannten Zange dadurch gelöst, daß an zumindest einem der Maulteile ein Adapter lösbar befestigbar ist, der eine sich zumindest über einen Teilbereich des einen Maulteiles, an dem er befestigbar ist, erstreckende stumpfe Stützfläche aufweist, die sich bei befestigtem Adapter beim Schließen der Maulteile auf dem anderen Maulteil flächig abstützt, so daß die Schneiden beim Schließen der Maulteile nicht mehr schneidend zusammenwirken können.

Erfindungsgemäß wird die Aufgabe hinsichtlich der eingangs an zweiter Stelle genannten Zange dadurch gelöst, daß an zumindest einem der Maulteile ein Adapter lösbar befestigbar ist, der eine Kante oder eine Schneide aufweist, die beim Schließen der Maulteile mit einer Kante des anderen Maulteiles oder mit einer Schneide eines am anderen Maulteil lösbar befestigten zweiten Adapters schneidend zusammenwirkt.

Die erfindungsgemäße Zange unterscheidet sich von den bekannten reinen Schneidzangen bzw. reinen Faßzangen dadurch, daß diese, falls die Maulteile als Schneidwerkzeuge ausgebildet sind, nach Anbringen des erfindungsgemäß vorgesehenen Adapters als Faßzange verwendbar ist und bei abgenommenem Adapter als Schneidzange arbeitet bzw. falls die Maulteile der Zange als Faßwerkzeuge ausgebildet sind, nach dem Anbringen des Adapters als Schneidzange arbeitet. Die erfindungsgemäße Zange hat somit abwechselnd die Funktion einer Schneidzange und einer Faßzange.

Sind die Maulteile der erfindungsgemäßen Zange als Schneidwerkzeuge ausgebildet, wird bei einer Gewebeentnahme die erfindungsgemäße Zange zunächst ohne den Adapter mit den Maulteilen in das Operationsgebiet geführt, um das zu entfernende Gewebe abzutrennen bzw. durchzuschneiden. Ist das Gewebestück abgetrennt, wird die Zange vom Operationsgebiet abgezogen. Dann wird der erfindungsgemäß vorgesehene Adapter an zumindest einem der Maulteile befestigt. Dazu muß der Arzt die Zange nicht aus der Hand legen sondern kann die Zange weiter in derselben Hand behalten und mit der anderen Hand den Adapter an dem Maulteil befestigen. Dies kann aus Sterilitätsgründen auch eine andere Person durchführen, wobei der Arzt die Zange entsprechend hält, ohne jedoch dabei die Zange loszulassen. Ist der Adapter befestigt, kann dann die Zange mit den Maulteilen wieder in das Operationsgebiet eingeführt werden, um nun mit den durch den Adapter zu Faßwerkzeugen umfunktionierten Maulteilen das abgetrennte Gewebe zu erfassen und aus dem Operationsgebiet zu entfernen. Da nun die Maulteile als reine Faßwerkzeuge arbeiten, braucht der Arzt seine Handkraft nicht vorsichtig zu dosieren, da nicht die Gefahr besteht, daß das gefaßte Gewebestück zertrennt wird.

Bei einer solchen Zange, bei der die Maulteile als Schneidwerkzeuge ausgebildet sind, besteht der Vorteil des Adapters darin, daß zwischen der stumpfen Stützfläche des Adapters und dem anderen Maulteil, auf der sich diese Stützfläche abstützt, das abgetrennte Gewebe sicher gefaßt werden kann, ohne dabei zertrennt zu werden. Da die Schneiden der Maulteile bei befestigtem Adapter beim Schließen der Maulteile nicht miteinander in Wirkungseingriff kommen, wenn der Adapter an dem einen Maulteil befestigt ist, wird die Gefahr ausgeschlossen, daß das Gewebe beim Fassen in unerwünschter Weise durch die scharfen Schneiden durchtrennt wird.

Ärzte arbeiten bei an sich ähnlichen Krankheitsbildern je nach Ausprägung der Krankheit und abhängig vom Patienten mit verschiedenen Behandlungsmethoden und Operationstechniken. Als Beispiel sei die Behandlung einer Zyste im HNO-Bereich angesprochen. Manchmal wird eine Zyste nicht ganz entfernt, sondern es wird nur geschnitten, und die auslaufende Flüssigkeit wird abgesaugt. Dazu ist eine Schneidezange notwendig. In manchen Fällen soll die gesamte Zyste entfernt werden, wozu eine Faßzange notwendig ist.

Die Erfindung ermöglicht nun, beide Behandlungsmethoden bzw. Operationsmethoden mit ein und derselben Zange unter Zuhilfenahme des Adapters durchzuführen.

Sind die Maulteile der erfindungsgemäßen Zange als Faßwerkzeuge zum Fassen von Gewebe ausgebildet, wird zum Abtrennen des Gewebes zuerst der zumindest eine Adapter an einem der Maulteile befestigt, der die Maulteile zu Schneidwerkzeugen umfunktioniert. Zum Entnehmen des Gewebes wird dann der Adapter abgenommen. Da die Vorgänge des Abtrennens und des Entfernens des Gewebes mit einer Zange durchgeführt werden können, muß sich der Arzt nur noch an die Bewegungscharakteristik einer einzigen Zange gewöhnen. Auch der Kostenaufwand der erfindungsgemäßen Zange ist auf vorteilhafte Weise reduziert, da anstelle von zwei kompletten Schneid- und Faßzangen nur noch eine Zange mit Griffelementen, Schaft und Betätigungselementen erforderlich ist, während der Adapter als einfaches Zusatzelement wesentlich kostengünstiger herstellbar ist als eine zweite komplette Zange.

Im Rahmen der Erfindung kann es auch vorgesehen sein, für beide Maulteile einen Adapter vorzusehen.

Somit wird die Aufgabe vollständig gelöst.

In einer weiteren bevorzugten Ausgestaltung der Zange, deren Maulteile Schneiden aufweisen, erstreckt sich die Stützfläche über etwa die gesamte Länge der Schneide des einen Maulteiles, an dem der Adapter befestigbar ist.

Diese Maßnahme hat den Vorteil, daß mit dem gesamten Bereich der Maulteile, der ohne den Adapter schneidend wirkt, das Gewebestück bei befestigtem Adapter gefaßt werden kann.

Weiterhin ist es dabei bevorzugt, daß die Stützfläche bei befestigtem Adapter über die Schneide des Maulteiles, an dem der Adapter befestigt ist, hinausragt.

Durch diese Maßnahme wird auf vorteilhafte Weise sicher gewährleistet, daß im befestigten Zustand des Adapters und beim Schließen der Maulteile die Schneiden nicht miteinander in Wirkungseingriff kommen können. Das Gewebe kann beim Fassen nicht zertrennt werden, da die über die Schneide des einen Maulteils hinausragende Stützfläche schon mit dem anderen Maulteil in Berührung tritt, bevor die beiden Schneiden miteinander in Wirkungseingriff kommen können.

In einem bevorzugten Ausführungsbeispiel ist das eine Maulteil beweglich und das andere Maulteil unbeweglich, wobei das bewegliche Maulteil zum Abtrennen des Gewebes in das unbewegliche Maulteil eingreift, und der Adapter ist an dem beweglichen Maulteil befestigbar.

Bei einer derartigen Ausbildung der Maulteile der erfindungsgemäßen Zange, die bei dieser Bauweise der Maulteile als durchschneidend bezeichnet wird, ist das bewegliche Maulteil, das in das unbewegliche Maulteil zum Abtrennen des Gewebes eingreift, schmaler ausgebildet als das unbewegliche Maulteil. Dadurch, daß der Adapter an dem schmaleren beweglichen Maulteil befestigbar ist, wird der Vorteil erzielt, daß die Gesamtbreite der Zange im Bereich der Maulteile durch den Adapter nicht vergrößert wird, was insbesondere bei besonders schmal bauenden Zangen wünschenswert ist, die in Operationsgebieten eingesetzt werden, die nur äußerst schwer zugänglich sind. Bei der weit verbreiteten minimal invasiven Chirurgie kann die Zange in beiden Funktionszuständen (also mit oder ohne Adapter) durch ein und dasselbe Endoskop oder denselben Trokar bewegt werden.

Dabei ist es bevorzugt, wenn das bewegliche Maulteil auf einer dem unbeweglichen Maulteil zugewandten Seite konkav ausgebildet ist.

Diese Maßnahme hat einerseits den Vorteil, daß an den Rändern des beweglichen Maulteiles besonders scharfe Schneidkanten ausgebildet werden können, wodurch die Funktion der erfindungsgemäßen Zange zum Abtrennen des Gewebes verbessert ist, andererseits kann das abgetrennte Gewebe in der Wölbung beim Fassen des Gewebestückes gefangen werden, so daß auch die Funktion der erfindungsgemäßen Zange als Faßzange dadurch verbessert ist.

In einer weiteren bevorzugten Ausgestaltung der Erfindung weist der Adapter Rastmittel auf, die mit entsprechenden Rastmitteln des einen Maulteiles, an dem der Adapter befestigbar ist, verrastbar sind.

Dadurch wird vorteilhafterweise eine einfach herstellbare und leicht lösbare Befestigungsmöglichkeit zwischen dem Adapter und dem einen Maulteil geschaffen, die einerseits eine sichere Befestigung des Adapters gewährleistet, so daß sich dieser nicht unerwünscht im Operationsgebiet von dem Maulteil ablöst, andererseits eine leichte und schnelle Handhabung beim Befestigen und Lösen des Adapters ermöglicht.

In einer weiteren bevorzugten Ausgestaltung der Erfindung weist der Adapter einen U-förmigen Körper auf.

Diese Ausgestaltung hat den Vorteil, daß sich der Adapter bequem zwischen zwei Fingern einer Hand halten läßt und sich leicht mit seinem offenen Ende auf ein Maulteil aufschieben oder aufstecken läßt, so daß die Handhabung der erfindungsgemäßen Zange weiter verbessert wird.

Dabei ist es bevorzugt, wenn zwei Schenkel des U-förmigen Körpers durch einen Steg miteinander verbunden sind, wobei der Steg bei befestigtem Adapter das Maulteil, an dem der Adapter befestigbar ist, übergreift.

Durch den Steg wird auf vorteilhafte Weise verhindert, daß sich die beiden Schenkel des U-förmigen Körpers beim Befestigen an dem einen Maulteil oder im befestigten Zustand beim Fassen oder Schneiden des Gewebes unerwünscht auseinanderbiegen. Der Adapter weist somit eine hohe Stabilität auf, die die Betriebssicherheit der erfindungsgemäßen Zange erhöht.

In einer weiteren bevorzugten Ausgestaltung der Erfindung weist das Maulteil, an dem der Adapter befestigbar ist, eine in Längsrichtung des Maulteiles verlaufende umfänglich angeordnete Nut auf, in die entsprechende Vorsprünge des Adapters eingreifen.

Diese Maßnahme hat den Vorteil, daß sich der Adapter an dem Maulteil immer in der gleichen Lage positionieren läßt. Darüber hinaus ist der Adapter durch die in die Nut eingreifenden Vorsprünge beim Schließen der Maulteile zum Fassen bzw. Schneiden des Gewebestückes gegen ein unerwünschtes Verschieben senkrecht zur Schließrichtung der Maulteile gesichert.

In einer weiteren bevorzugten Ausgestaltung ist der Adapter einstückig aus Metall gefertigt.

Durch die einstückige Ausbildung des Adapters ist eine konstruktiv besonders einfache und kostengünstige Herstellung des Adapters möglich. Dadurch daß der Adapter aus Metall gefertigt ist, läßt er sich besonders gut sterilisieren und kann somit mehrfach verwendet werden.

Weitere Vorteile ergeben sich aus der nachfolgenden Beschreibung der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach näher erläutert. Es zeigen:
- Fig. 1: eine medizinische Zange in Seitenansicht;
- Fig. 2: einen Schnitt durch die Längsmittelebene der beiden als Schneidwerkzeuge ausgebildeten Maulteile der medizinischen Zange von Fig. 1 in vergrößertem Maßstab, wobei auf ein Maulteil ein Adapter aufgesetzt ist;
- Fig. 3: einen Schnitt durch die Maulteile entlang der Linie III-III von Fig. 2;
- Fig. 4: einen der Fig. 3 entsprechenden Schnitt durch die beiden Maulteile ohne Adapter;
- Fig. 5: eine Unteransicht des Adapters;
- Fig. 6: einen Schnitt durch den Adapter entlang der Linie VI-VI von Fig. 5;
- Fig. 7: eine Draufsicht auf den Adapter;
- Fig. 8: einen Schnitt durch den Adapter entlang der Linie VIII-VIII von Fig. 7;
- Fig. 9: eine teilweise geschnittene Draufsicht auf das bewegliche Maulteil mit dem daran befestigten Adapter;
- Fig. 10: ein weiteres Ausführungsbeispiel der Erfindung in einem der Fig. 3 entsprechenden Schnitt;
- Fig. 11: ein weiteres Ausführungsbeispiel der Erfindung in einem ebenfalls der Fig. 3 entsprechenden Schnitt;
- Fig. 12: ein Ausführungsbeispiel einer Zange, deren Maulteile als Faßwerkzeuge ausgebildet sind; und
- Fig. 13: ein weiteres Ausführungsbeispiel einer Zange, deren Maulteile als Faßwerkzeuge ausgebildet sind.

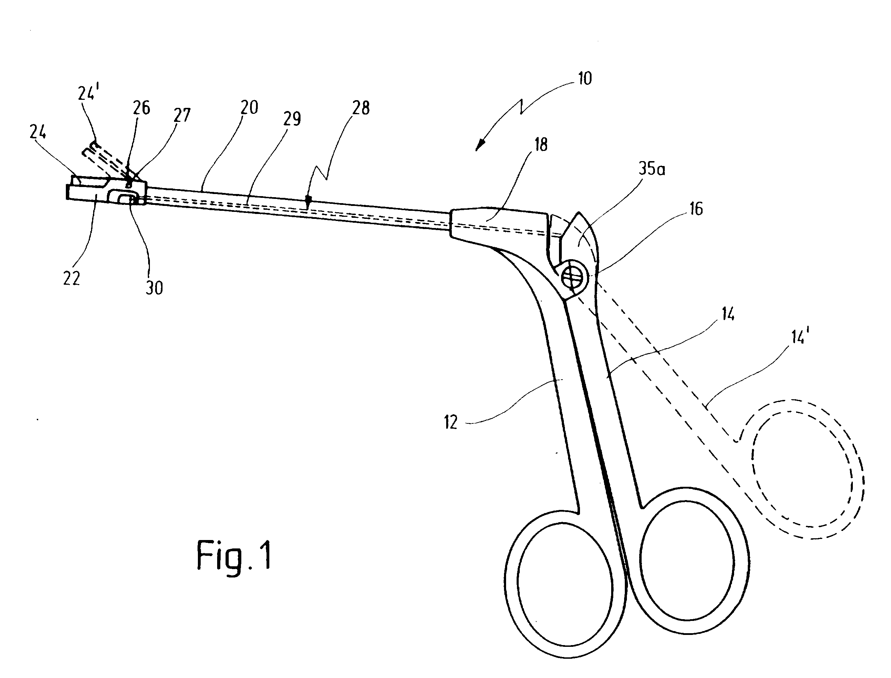

Fig. 1 zeigt eine insgesamt mit dem Bezugszeichen 10 bezeichnete medizinische Zange. Die Zange 10 wird zur Gewebeentnahme im menschlichen oder tierischen Körper verwendet.

Am patientenfernen Ende der Zange 10 ist ein unbewegliches Griffelement 12 sowie ein bewegliches Griffelement 14 vorgesehen, die über ein Scharniergelenk 16 miteinander verbunden sind, und zwar ist das bewegliche Griffelement 14 um das Scharniergelenk 16 als Drehachse in eine Offenlage verschwenkbar, die mit 14' gestrichelt dargestellt ist. Von einem Endbereich 18 des unbeweglichen Griffelementes 12 führt ein langgestreckter rohrförmiger Schaft 20 ab. Die Griffteile 12 und 14 sowie der Schaft 20 sind aus einem Metall oder einem Kunststoff mit vergleichbaren Eigenschaften hergestellt.

Am patientennahen Ende des Schaftes 20 ist ein unbewegliches Maulteil 22 sowie ein bewegliches Maulteil 24 angeordnet. Das unbewegliche Maulteil 22 ist mit dem Schaft 20 fest verbunden. Das bewegliche Maulteil 24 ist an dem unbeweglichen Maulteil 22 über einen Achszapfen 26 als Drehachse 27 verschwenkbar befestigt.

Das bewegliche Maulteil 24 ist über ein Betätigungselement 28 kraftschlüssig mit dem beweglichen Griffelement 14 verbunden. Das Betätigungselement 28 weist einen Drahtkörper 29 auf, der an seinem patientennahen Ende einen Zapfen 30 aufweist, der sich quer zu dem Drahtkörper 29 erstreckt. Der Zapfen 30 ist zusammen mit dem Drahtkörper 29 aus einem Vollmaterial herausgearbeitet, und zwar aus einem gehärteten Stahl.

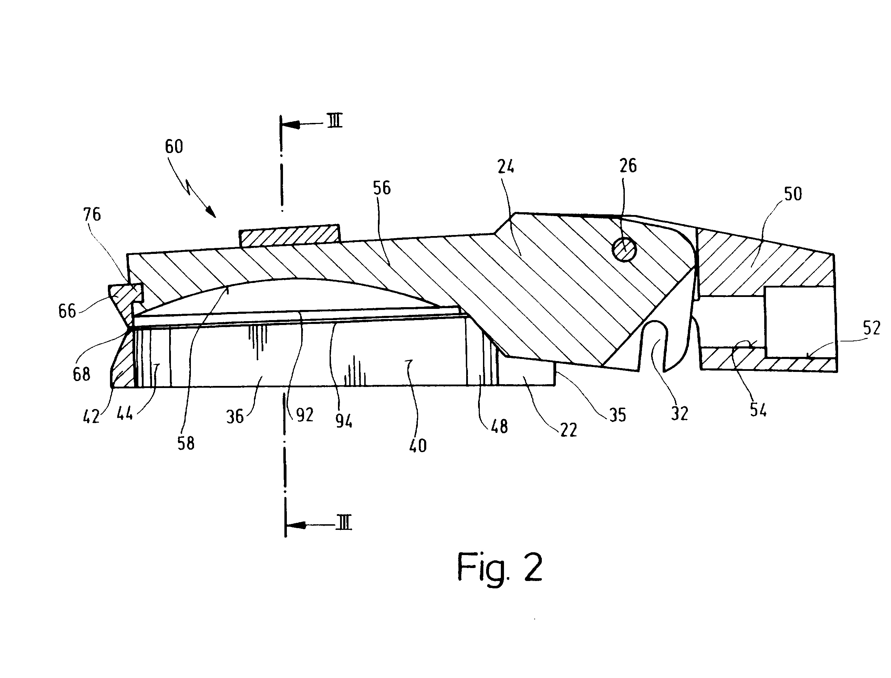

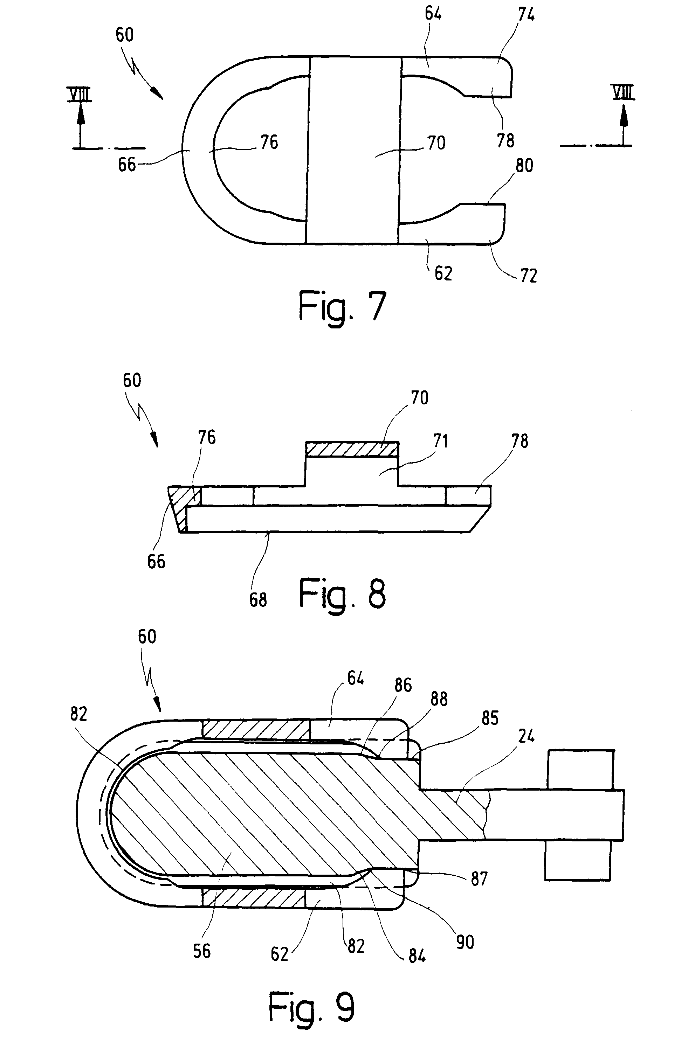

Der Zapfen 30 greift in eine entsprechende Aussparung 32 (vgl. Fig. 2) des beweglichen Maulteiles 24 ein, wobei die Aussparung 32 in einem verbreiterten massiven Abschnitt 33 (vgl. Fig. 9) des beweglichen Maulteiles 24 angeordnet ist, der in einer Ausnehmung 35 (vgl. Fig. 2) des unbeweglichen Maulteiles 22 aufgenommen ist. Das Betätigungselement 28 verläuft von dem beweglichen Maulteil 24 ausgehend in dem Schaft 20 durch den Endbereich 18 des unbeweglichen Griffelementes 12 bis zu einem Endbereich 35a des beweglichen Griffelementes 14, wo es durch hier nicht dargestellte Befestigungsmittel befestigt ist. Beim Schließen der Griffelemente 12, 14 (gestrichelte Lage) bewegt sich das geöffnete Maulteil 24 (gestrichelte Lage) auf das unbewegliche Maulteil 22 zu und läuft durch dieses hindurch.

In den Figuren 2 bis 4 sind die Maulteile 22 und 24 der medizinischen Zange 10 in vergrößertem Maßstab dargestellt. Das unbewegliche Maulteil 22 wird aus zwei etwa parallel zueinander verlaufenden seitlichen Schenkeln 36 und 38 gebildet, wobei in der Schnittdarstellung der Fig. 2 eine glatte gerade Innenwand 40 des in Richtung zum patientennahen Ende gesehenen rechten Schenkels 36 zu sehen ist.

Am patientennahen Ende laufen die Schenkel 36 und 38 etwa halbkreisförmig zu einem Scheitelabschnitt 42 zusammen. Die Innenwand 40 des Schenkels 36 geht somit stetig über eine halbkreisförmig gekrümmte Innenwand 44 in eine wiederum gerade verlaufende Innenwand 46 des Schenkels 38 über. Die Innenwände 40, 44 und 46 sind in vertikaler Richtung gerade ausgebildet, so daß im patientennahen Bereich ein im wesentlichen U-förmiger freier Raum zwischen den Schenkeln 36 und 38 gebildet wird.

In einem Mittelabschnitt 48 ist der Schenkel 36 nach innen gekrümmt, das gleiche gilt für den Schenkel 38, ohne daß die Schenkel 36 und 38 dort miteinander verbunden sind. Erst am äußeren patientenfernen Ende sind die Schenkel 36 und 38 durch einen Endabschnitt 50 einstückig miteinander verbunden. Der Bereich zwischen den Innenwänden 40, 44 und 46 ist in vertikaler Richtung durchgehend offen.

In dem Endabschnitt 50 ist eine Bohrung 52 vorgesehen, mit der das unbewegliche Maulteil 22 auf den Schaft 20 aufsteckbar und beispielsweise durch Verlöten schaftfest befestigbar ist. Die Bohrung 52 setzt sich in einer weiteren Bohrung 54 kleineren Durchmessers fort, die zur Durchführung des Betätigungselementes 28 vorgesehen ist, das mit dem Zapfen 30 in der Aussparung 32 des unbeweglichen Maulteiles 24 unverlierbar befestigbar ist.

Das bewegliche Maulteil 24 ist zwischen den Schenkeln 36 und 38 des unbeweglichen Maulteiles 22 angeordnet und als massiver Körper ausgebildet, dessen Umfangskontur in einem patientennahen Bereich 56 des Maulteiles 24 der durch die Innenwände 40, 44 und 46 des unbeweglichen Maulteiles 22 bestimmten Kontur entspricht.

Eine dem unbeweglichen Maulteil 22 zugewandte untere Fläche 58 des beweglichen Maulteiles 24 ist konkav ausgebildet.

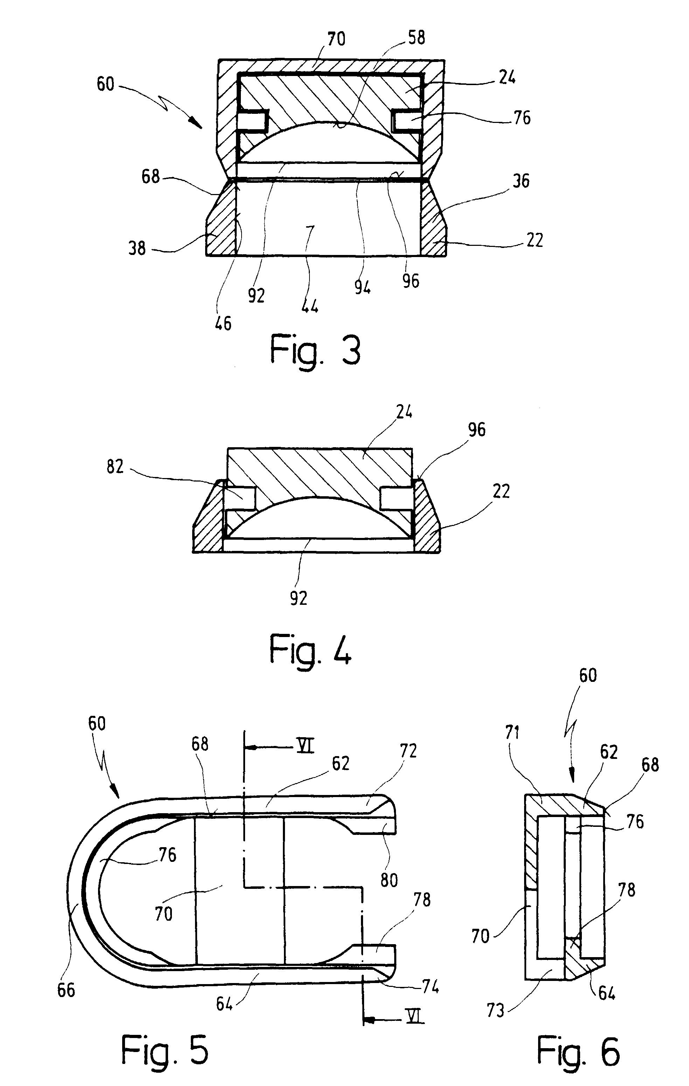

In den Figuren 2 und 3 ist an der medizinischen Zange 10, und zwar genauer an dem beweglichen Maulteil 24, ein allgemein mit 60 bezeichneter Adapter befestigt, durch den die Zange 10 als Faßzange arbeitet, wie dies in Fig. 3 dargestellt ist. Ohne den Adapter 60 arbeitet die Zange 10 als Schneidzange, wie dies in Fig. 4 dargestellt ist. Daraus ist ersichtlich, daß das bewegliche Maulteil 24, ohne den Adapter 60, durch das unbewegliche Maulteil 22 hindurch laufen kann, die Zange 10 somit als durchschneidende Zange arbeitet.

Der Adapter 60 ist an dem beweglichen Maulteil 24 lösbar befestigbar, d.h. der Adapter 60 ist auf das bewegliche Maulteil 24 aufsteckbar und von diesem wieder abnehmbar.

Bevor auf die Funktion des Adapters 60 im Zusammenwirken mit den Maulteilen 22 und 24 eingegangen wird, soll zunächst anhand der Figuren 5 bis 8 die Ausgestaltung des Adapters 60 näher beschrieben werden.

Der Adapter 60 ist insgesamt als metallischer U-förmiger Körper ausgebildet, mit zwei Schenkeln 62 und 64, die über einen etwa halbkreisförmig ausgebildeten Abschnitt 66 miteinander einstükkig verbunden sind. Die Schenkel 62 und 64 verjüngen sich einseitig keilförmig zur Unterseite (Fig. 5 und 6) des Adapters 60 hin zu einer schmalen Stützfläche 68, die auf der Unterseite des Adapters 60 ebenfalls U-förmig über den gesamten Körper des Adapters 60 verläuft.

Auf der Oberseite (Fig. 7) des Adapters 60 verbindet ein Steg 70 in Form einer ebenen rechteckigen Platte die beiden Schenkel 62 und 64 einstückig über vertikale Seitenwände 71 und 73 miteinander, während freie Enden 72 und 74 der Schenkel 62 und 64 unverbunden sind, so daß diese elastisch biegbar sind.

Weiterhin weist der halbkreisförmige Abschnitt 66 des Adapters 60 einen ebenfalls etwa halbkreisförmig gekrümmten nach innen gerichteten Vorsprung 76 auf.

Die freien Enden 72 und 74 sind mit Rastmitteln 78 und 80 ebenfalls in Form von nach innen gerichteten Vorsprüngen versehen, die sich über einen Teilbereich der freien Enden 72 und 74 der Schenkel 62 und 64 erstrecken und in derselben Ebene liegen wie der halbkreisförmige Vorsprung 76.

In Fig. 9 ist der Adapter 60 an dem beweglichen Maulteil 24 befestigt dargestellt.

Der Adapter 60 ist vom patientennahen Ende her auf das bewegliche Maulteil 24 aufgesteckt bzw. aufgeschoben, wobei die Rastmittel 78 und 80 in Form von Vorsprüngen und der Vorsprung 76 in eine in dem beweglichen Maulteil 24 ausgesparte Nut 82 eingreifen, die bezüglich der Höhe des beweglichen Maulteiles 24 etwa mittig angeordnet ist und umfänglich um den gesamten patientennahen Bereich 56 des beweglichen Maulteiles 24 U-förmig verläuft. Im befestigten Zustand übergreift der Steg 70 das bewegliche Maulteil 24 auf dessen Oberseite, wie in Fig. 3 zu erkennen ist.

Patientenfernere Abschnitte 85 und 87 der Nut 82 sind etwas tiefer in das bewegliche Maulteil 24 eingeschnitten und gehen über abgeschrägte Bereiche 84 und 86 in den übrigen Teil der Nut 82 über. Die patientenfernen Enden der Bereiche 84 und 86 bilden Rastmittel 88 und 90 des beweglichen Maulteiles 24.

Zum Aufstecken bzw. Aufschieben des Adapters 60 auf das bewegliche Maulteil 24 werden dessen freie Enden 72 und 74 vom patientennahen Ende des beweglichen Maulteiles 24 aus mit den Vorsprüngen 78 und 80 in die Nut 82 eingesetzt. Anschließend wird der Adapter 60 in Richtung zum patientenfernen Ende des beweglichen Maulteiles 24 hin geschoben, bis die Vorsprünge 78 und 80 des Adapters 60 die abgeschrägten Bereiche 84 und 86 überlaufen haben und in den Abschnitten 85 und 87 der Nut 82 zum Liegen kommen. In diesem Zustand bilden die abgeschrägten Bereiche Sperren gegen Abziehen des Adapters, dieser ist somit verrastet. Das Verrasten geschieht dadurch, daß die freien Enden 72 und 74 des Adapters 60 beim Aufschieben in der Nut 82 zunächst etwas gespreizt werden und nach Überlaufen der abgeschrägten Bereiche 84 und 86 in den tiefer eingeschnittenen Abschnitten 85 und 87 der Nut 82 zu liegen kommen. Zum Abnehmen des Adapters 60 wird dieser in Richtung zum patientennahen Ende des Maulteiles 24 hin vom Maulteil 24 wieder abgezogen.

Anhand der Figuren 3 und 4 wird im folgenden die Funktion der medizinischen Zange 10 als Schneid- und Faßzange erläutert.

In Fig. 4 ist der Fall dargestellt, daß die Zange 10 als Schneidzange zum Abtrennen von Gewebe verwendet wird. In diesem Fall wirken die Maulteile 22 und 24 als Schneidwerkzeuge zusammen.

Dazu weist das bewegliche Maulteil 24 auf seiner unteren, d.h. dem unbeweglichen Maulteil 22 zugewandten Seite eine U-förmige Schneide 92 in Form einer scharfen Kante auf, die mit einer entsprechenden Schneide 94 des unbeweglichen Maulteiles 22 zusammenwirkt, die durch die obere Kante der Innenwände 40, 44 und 46 des unbeweglichen Maulteiles 22 gebildet wird. Die Schneiden 92 und 94 verlaufen entsprechend der Ausgestaltung der Maulteile 22 und 24 U-förmig. Zum Abtrennen bzw. Durchschneiden von Gewebe werden die Maulteile 22 und 24 über die Griffelemente 12 und 14 geschlossen, so daß das bewegliche Maulteil 24 beim Schließen in das unbewegliche Maulteil 22 eingreift, wobei die Schneiden 92 und 94 aneinander vorbeigeführt werden, wodurch die Schneidwirkung zustande kommt. Bei einer derartigen Ausbildung der Maulteile 22, 24 wird die Zange 10 als durchschneidende Zange bezeichnet.

In Fig. 3 ist dagegen der Fall dargestellt, daß die Zange 10 als Faßzange arbeitet, indem der Adapter 60 an dem beweglichen Maulteil 24 befestigt ist. Der Adapter 60 ist bezüglich des unbeweglichen Maulteiles 24 so ausgebildet, daß dessen Stützfläche 68 die Schneide 92 des beweglichen Maulteiles 24 in Richtung unbewegliches Maulteil 22 überragt. Die Stützfläche 68 erstreckt sich über etwa die gesamte Länge der Schneide 92 des beweglichen Maulteiles 24, so daß der gesamte schneidend wirkende Bereich der Maulteile 22 und 24 bei befestigtem Adapter 60 als wirksamer Bereich zum Fassen ausgenutzt wird. Beim Schließen der Maulteile 22 und 24 stößt nun die Stützfläche 68 des Adapters 60 flächig, d.h. stumpf auf einen auf der Oberseite, d.h. der dem beweglichen Maülteil 24 zugewandten Seite des unbeweglichen Maulteiles 22 ebenfalls als Stützfläche flächig ausgebildeten Rand 96, ohne daß die Schneiden 92 und 94 miteinander in Wirkungseingriff kommen können. Die Schneiden 92 und 94 sind in der geschlossenen Lage der Maulteile 22 und 24 voneinander beabstandet. In Fig. 3 ist bereits die maximale Schließlage der Maulteile 22 und 24 dargestellt, wenn der Adapter 60 an dem beweglichen Maulteil 24 befestigt ist.

Zwischen der Stützfläche 68 des Adapters 60 und dem Rand 96 des unbeweglichen Maulteiles 22 kann nun das zuvor abgetrennte Gewebe gefaßt und zum Entnehmen aus dem Körper sicher festgehalten werden, ohne dabei zertrennt zu werden.

Bei dem Ausführungsbeispiel der Fig. 3 verjüngen sich die Schenkel 36 und 38 des unbeweglichen Maulteiles 22 auf ihrer Außenseite keilförmig zu dem Rand 96 hin, während sich die Schenkel 62 und 64 des Adapters 60, wie bereits erwähnt, auf ihrer Außenseite zu der Stützfläche 68 verjüngen.

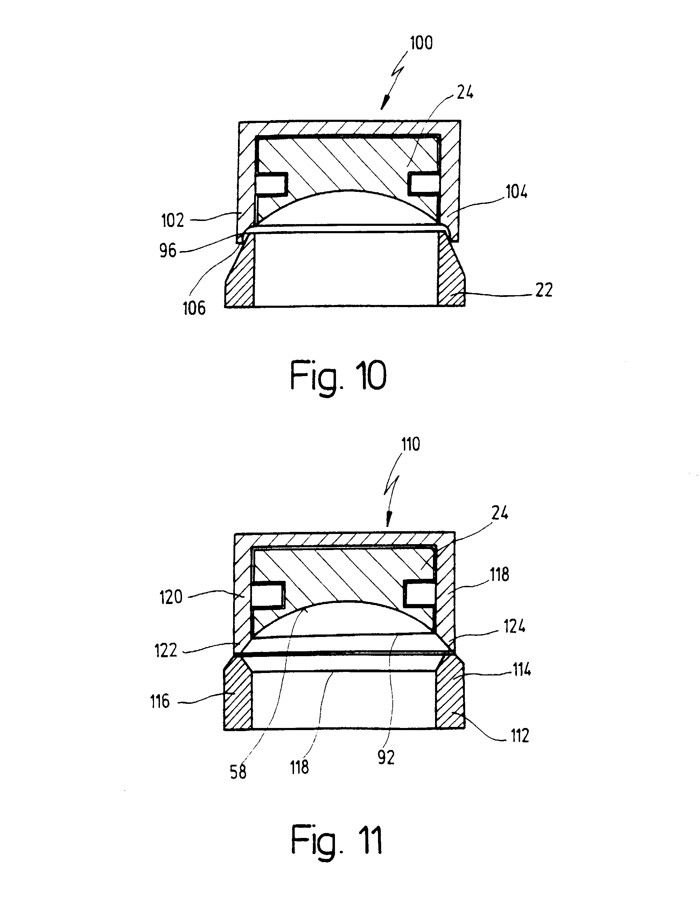

In Fig. 10 ist als weiteres Ausführungsbeispiel ein gegenüber dem Adapter 60 abgewandelter Adapter 100 dargestellt, der auf dem unverändert ausgestalteten beweglichen Maulteil 24 lösbar befestigbar ist. Schenkel 102 und 104 des Adapters 100 weisen eine untere Stützfläche 106 auf, die sich nicht auf dem Rand 96 des unbeweglichen Maulteiles 22 abstützt, sondern den Rand 96 übergreift und sich auf den abgeschrägten Seiten des Maulteiles 22 abstützt. Dadurch ist eine größere Faßfläche geschaffen.

In Fig. 11 ist ein weiteres Ausführungsbeispiel der Erfindung dargestellt, bei dem ein Adapter 110 sowie ein unbewegliches Maulteil 112 gegenüber dem Adapter 60 und dem unbeweglichen Maulteil 22 der Figuren 1 bis 9 abgewandelt ausgestaltet ist.

Das unbewegliche Maulteil 112 weist Schenkel 114 und 116 auf, die auf ihrer dem beweglichen Maulteil 24 zugewandten oberen Seite innen abgeschrägt ausgebildet sind, so daß eine umlaufende Kante 118 eine Schneidkante des unbeweglichen Maulteiles 112 bildet, die mit der Schneidkante 92 des beweglichen Maulteiles 24 zusammenwirkt.

Der Adapter 110 weist Schenkel 118 und 120 auf, die an dem unbeweglichen Maulteil 112 zugewandten Enden 122 und 124 innenseitig entsprechend der Krümmung der innenliegenden Fläche 58 des beweglichen Maulteiles 24 gekrümmt sind. Dadurch ist eine relativ kleine Faßfläche geschaffen, über die zum Beispiel eine Zyste gefaßt und anschließend abgerissen werden kann.

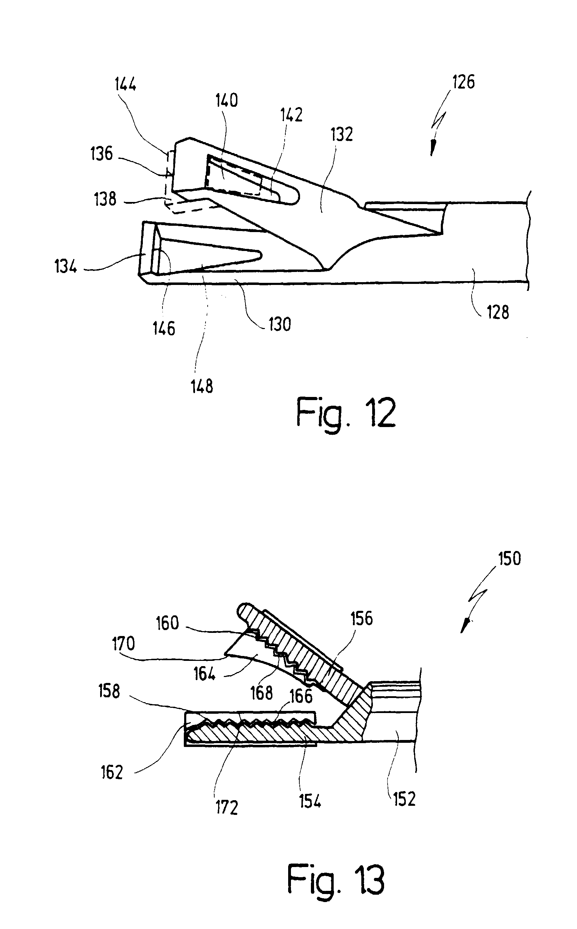

In Fig. 12 ist gemäß einem weiteren Ausführungsbeispiel das patientennahe Ende einer medizinischen Zange 126 dargestellt, mit einem Schaft 128, an dessen patientennahem Ende zwei als Faßwerkzeuge ausgebildete Maulteile 130 und 132 befestigt sind und über hier nicht dargestellte Griffelemente am patientenfernen Ende der Zange 126 relativ zueinander beweglich sind, wobei das Maulteil 130 schaftfest und das Maulteil 132 beweglich ist. Die Maulteile 130 und 132 weisen an ihrem patientennahen Ende Flächen 134 und 136 auf, die beim Schließen flächig aneinanderstoßen, so daß die Maulteile 130 und 132 beim Schließen bereits abgetrenntes Gewebe fassen können, ohne dieses zu zertrennen.

Für das bewegliche Maulteil 132 ist ein gestrichelt dargestellter Adapter 138 vorgesehen, der an dem Maulteil 132 lösbar befestigbar ist.

Dazu weist der Adapter 138 einen Abschnitt 140 auf, der in ein in dem Maulteil 132 vorgesehenes Fenster 142 beispielsweise über ein System von hier nicht dargestellten Nuten und Vorsprüngen einrastbar ist, so daß der Adapter 138 unverlierbar an dem Maulteil 132 gehalten wird.

Der Adapter 138 weist eine Kante 144 auf, die über die Fläche 136 des Maulteils 132 vorragt und vom patientennahen Ende aus gesehen hinter dieser angeordnet ist, so daß sie mit einer Kante 146 des unbeweglichen Maulteils 130 beim Schließen der Maulteile 130 und 132 zum Abtrennen von Gewebe schneidend zusammenwirkt. Beim Schließen der Maulteile 130 und 132 greift der Adapter 138 in ein Fenster 148 des unbeweglichen Maulteils 130 ein, derart, daß die Maulteile 130 und 132 bei befestigtem Adapter 138 als durchschneidende Schneidwerkzeuge zusammenwirken.

Schließlich ist in Fig. 13 das patientennahe Ende einer Zange 150 dargestellt, die einen Schaft 152 aufweist, an dessen patientenahem Ende ein unbewegliches Maulteil 154 und ein bewegliches Maulteil 156 angeordnet sind.

Die Maulteile 154 und 156 sind als Faßwerkzeuge ausgebildet, und zwar weisen die Maulteile 154 und 156 Zahnungen 158 und 160 auf, die beim Schließen der Maulteile 154 und 156 ineinandergreifen und zwischen denen ein zuvor abgetrenntes Gewebestück gefaßt werden kann. Für das Maulteil 154 ist ein lösbar daran befestigbarer Adapter 162 und für das Maulteil 156 ein lösbar daran befestigbarer Adapter 164 vorgesehen. Zur unverlierbaren Befestigung der Adapter 162 und 164 weisen diese entsprechende Zahnungen 166 und 168 auf, die im aufgesteckten Zustand der Adapter 162 und 164 in die Zahnungen 158 und 160 der Maulteile 154 und 156 eingreifen und dadurch mit diesen verrastet sind. Der Adapter 164 weist eine Schneide 170 auf, die im befestigten Zustand des Adapters 164 seitlich an dem beweglichen Maulteil 156 angeordnet ist. Die Schneide 170 wirkt beim Schließen der Maulteile 154 und 156 mit einer äußeren Kante 172 des Adapters 162 zum Abtrennen von Gewebe schneidend, und zwar scherenartig schneidend zusammen.

## Patentansprüche

1. Medizinische Zange zur Gewebeentnahme im menschlichen oder tierischen Körper, mit zwei Maulteilen (22, 24; 112) am patientennahen Ende, die über Griffelemente (12, 14) am patientenfernen Ende relativ zueinander bewegbar sind, wobei die Maulteile (22, 24; 112) Schneiden (92, 94) aufweisen, die beim Schließen der Maulteile (22, 24; 112) schneidend zusammenwirken, dadurch gekennzeichnet, daß an zumindest einem der Maulteile (22, 24; 112) ein Adapter (60; 100; 110) lösbar befestigbar ist, der eine sich zumindest über einen Teilbereich des einen Maulteils (24), an dem er befestigbar ist, erstreckende stumpfe Stützfläche (68; 106) aufweist, die sich bei befestigtem Adapter (60; 100; 110) beim Schließen der Maulteile (22, 24; 112) auf dem anderen Maulteil (22; 112) flächig abstützt, so daß die Schneiden (90, 92) beim Schließen der Maulteile (22, 24; 112) nicht mehr schneidend zusammenwirken können.

2. Zange nach Anspruch 1, dadurch gekennzeichnet, daß sich die Stützfläche (68; 106) über etwa die gesamte Länge der Schneide (92, 94) des einen Maulteiles (24) erstreckt, an dem der Adapter (60; 100; 110) befestigbar ist.

3. Zange nach Anspruch 1 oder 2, dadurch gekennzeichnet, daß die Stützfläche (68; 106) bei befestigtem Adapter (60; 100; 110) über die Schneide (92) des Maulteiles (24), an dem der Adapter (60; 100; 110) befestigbar ist, hinausragt.

4. Zange nach einem oder mehreren der Ansprüche 1 - 3, dadurch gekennzeichnet, daß eines der Maulteile (22, 24; 112) beweglich und das andere Maulteil (22) unbeweglich ist, wobei das bewegliche Maulteil (24) zum Abtrennen des Gewebes in das unbewegliche Maulteil (22) eingreift, und daß der Adapter (60; 100; 110) an dem beweglichen Maulteil (24) befestigbar ist.

5. Zange nach Anspruch 4, dadurch gekennzeichnet, daß das bewegliche Maulteil (24) auf einer dem unbeweglichen Maulteil (22; 122) zugewandten Seite konkav ausgebildet ist.

6. Medizinische Zange zur Gewebeentnahme im menschlichen oder tierischen Körper, mit zwei Maulteilen (130, 132; 154, 156) am patientennahen Ende, die über Griffelemente (12, 14) am patientenfernen Ende relativ zueinander bewegbar sind, wobei die Maulteile (130, 132; 154, 156) beim Schließen zum Fassen von Gewebe stumpf aufeinanderstoßen, dadurch gekennzeichnet, daß an zumindest einem der Maulteile (130, 132; 154, 156) ein Adapter (138; 162, 164) lösbar befestigbar ist, der eine Kante (144) oder eine Schneide (170) aufweist, die beim Schließen der Maulteile (130, 132; 154, 156) mit einer Kante (146) des anderen Maulteiles (130) oder mit einer Schneide (172) eines am anderen Maulteil (156) lösbar befestigten zweiten Adapters (164) schneidend zusammenwirkt.

7. Zange nach einem oder mehreren der Ansprüche 1 - 6, dadurch gekennzeichnet, daß der Adapter (60; 100; 110; 138; 162, 164) Rastmittel (78, 80) aufweist, die mit entsprechenden Rastmitteln (88, 90) des einen Maultteiles (24; 132; 154, 156), an dem der Adapter (60; 100; 110; 138; 162, 164) befestigbar ist, verrastbar sind.

8. Zange nach einem oder mehreren der Ansprüche 1 - 7, dadurch gekennzeichnet, daß der Adapter (60; 100; 110) einen U-förmigen Körper aufweist.

9. Zange nach Anspruch 8, dadurch gekennzeichnet, daß zwei Schenkel (62, 64; 102, 104; 118, 120) des U-förmigen Körpers durch einen Steg (70) miteinander verbunden sind, wobei der Steg (70) bei befestigtem Adapter (60; 100; 110) das Maulteil (24), an dem der Adapter (60; 100; 110) befestigbar ist, übergreift.

10. Zange nach einem oder mehreren der Ansprüche 1 - 9, dadurch gekennzeichnet, daß das Maulteil (24), an dem der Adapter (60; 100; 110) befestigbar ist, eine in Längsrichtung des Maulteiles (24) verlaufende und umfänglich angeordnete Nut (82) aufweist, in die entsprechende Vorsprünge (76) des Adapters (60; 100; 110) eingreifen.

11. Zange nach einem oder mehreren der Ansprüche 1 - 10, dadurch gekennzeichnet, daß der Adapter (60; 100; 110; 138; 162, 164) einstückig aus Metall gefertigt ist.

## Claims

1. A medical forceps for removal of tissue from the human or animal body, having two jaw parts (22, 24; 112) at the end nearest the patient which can be moved relative to one another via grip elements (12, 14) at the end remote from the patient, the jaw parts (22, 24; 112) having blades (92, 94) which coact in cutting fashion as the jaw parts (22, 24; 112) are closed, characterized in that there is releasably mounted on at least one of the jaw parts (22, 24; 112) an adapter (60; 100; 110) which has a blunt support surface (68; 106), extending at least over a portion of the one jaw part (24) on which it is mountable, which braces in planar fashion against the other jaw part (22; 112) as the jaw parts (22, 24; 112) are closed when the adapter (60; 100; 110) is mounted, so that the blades (90, 92) can no longer coact in cutting fashion as the jaw parts (22, 24; 112) are closed.

2. The forceps of claim 1, characterized in that the support surface (68; 106) extends over approximately the entire length of the blade (92, 94) of the one jaw part (24) on which the adapter (60; 100; 110) is mountable.

3. The forceps of claim 1 or 2, characterized in that when the adapter (60; 100; 110) is mounted, the support surface (68; 106) projects out beyond the blade (92) of the jaw part (24) on which the adapter (60; 100; 110) is mountable.

4. The forceps of one or more of Claims 1 through 3, characterized in that one of the jaw parts (22, 24; 112) is movable and the other jaw part (22) is immovable, the movable jaw part (24) engaging into the immovable jaw part (22) in order to detach the tissue, and that the adapter (60; 100; 110) is mountable on the movable jaw part (24).

5. The forceps of claim 4, characterized in that the movable jaw part (24) is of concave configuration on a side facing the immovable jaw part (22; 122).

6. A medical forceps for removal of tissue from the human or animal body, having two jaw parts (130, 132; 154, 156) at the end nearest the patient which can be moved relative to one another via grip elements (12, 14) at the end remove from the patient, the jaw parts (130, 132; 154, 156) butting against one another in blunt fashion upon closing in order to grasp tissue, characterized in that there is releasably mounted on at least one of the jaw parts (130, 132; 154, 156) an adapter (138; 162, 164) which has an edge (144) or a blade (170) which, as the jaw parts (130, 132; 154, 156) are closed, coacts in cutting fashion with an edge (146) of the other jaw part (130) or with a blade (172) of a second adapter (164) releasably mounted on the other jaw part (156).

7. The forceps of one or more of claims 1 through 6, characterized in that the adapter (60; 100; 110; 138; 162, 164) has snap-lock means (78, 80) which can be snap-locked to corresponding snap-lock means (88, 90) of the one jaw part (24; 132; 154, 156) on which the adapter (60; 100; 110; 138; 162, 164) is mountable.

8. The forceps of one or more of claims 1 through 7, characterized in that the adapter (60; 100; 110) has a U-shaped body.

9. The forceps of claim 8, characterized in that two limbs (62, 64; 102, 104; 118, 120) of the U-shaped body are joined together by a web (70), such that when the adapter (60; 100; 110) is mounted, the web (70) fits around the jaw part (24) on which the adapter (60, 100; 110) is mountable.

10. The forceps of one or more of claims 1 through 9, characterized in that the jaw part (24) on which the adapter (60; 100; 110) is mountable has a circumferentially arranged groove (82), running in the longitudinal direction of the jaw part (24), into which corresponding projections (76) of the adapter (60; 100; 110) engage.

11. The forceps of one or more of claims 1 through 10, wherein the adapter (60; 100; 110; 138; 162, 164) is made integrally of metal.

## Revendications

1. Pince médicale pour le prélèvement de tissus dans le corps humain ou animal, comportant deux parties de mâchoire (22, 24; 112) à l'extrémité proche du patient et qui sont déplaçables l'une par rapport à l'autre par des éléments formant poignée (12, 14) à l'extrémité éloignée du patient, les parties de mâchoire (22, 24 ; 112) présentant des lames (92, 94) qui coopèrent de manière coupante à la fermeture des parties de mâchoire (22, 24; 112), caractérisée en ce qu'à l'une au moins des parties de mâchoire (22, 24 ; 112) peut être fixé de manière non permanente un adaptateur (60 ; 100 ; 110) qui présente une surface d'appui (68 ; 106) émoussée, qui s'étend au moins sur une zone partielle d'une partie de mâchoire (24) à laquelle il est fixé et qui prend appui à plat sur l'autre partie de mâchoire (22 ; 112) à la fermeture des parties de mâchoire (22, 24 ; 112), lorsque l'adaptateur (60 ; 100 ; 110) est fixé, de sorte que les lames (90, 92) ne peuvent plus coopérer de manière coupante lorsque les parties de mâchoire (22, 24 ; 112) se ferment.

2. Pince selon la revendication 1, caractérisée en ce que la surface d'appui (68 ; 106) s'étend sensiblement sur toute la longueur de la lame (92, 94) d'une partie de mâchoire (24) à laquelle l'adaptateur (60 ; 100 ; 110) peut être fixé.

3. Pince selon la revendication 1 ou 2, caractérisée en ce que lorsque l'adaptateur (60 ; 100 ; 110) est fixé, la surface d'appui (68 ; 106) dépasse de la lame (92) de la partie de mâchoire (24) à laquelle l'adaptateur (60 ; 100 ; 110) peut être fixé.

4. Pince selon une ou plusieurs des revendications 1 à 3, caractérisée en ce que l'une des parties de mâchoire (22, 24 ; 112) est mobile et l'autre partie de mâchoire (22) est fixe, la partie de mâchoire mobile (24) s'engageant dans la partie de mâchoire fixe (22) pour séparer le tissu, et en ce que l'adaptateur (60 ; 100 ; 110) peut être fixé à la partie de mâchoire mobile (24).

5. Pince selon la revendication 4, caractérisée en ce que la partie de mâchoire mobile (24) est concave sur un côté tourné vers la partie de mâchoire fixe (22 ; 122).

6. Pince médicale pour le prélèvement de tissus dans le corps humain ou animal, comportant deux parties de mâchoire (130, 132 ; 154,156) à l'extrémité proche du patient et qui sont déplaçables l'une par rapport à l'autre par des éléments formant poignée (12, 14) à l'extrémité éloignée du patient, les parties de mâchoire (130, 132 ; 154, 156) se joignant bout à bout lors de la fermeture pour saisir un tissu, caractérisée en ce qu'à l'une au moins des parties de mâchoire (130, 132 ; 154, 156) peut être fixé de manière non permanente un adaptateur (138 ; 162, 164) qui présente une arête (144) ou une lame (170) qui, à la fermeture des parties de mâchoire (130, 132 ; 154, 156), coopère de manière coupante avec une arête (146) de l'autre partie de mâchoire (130) ou avec une lame (172) d'un deuxième adaptateur (164) fixé de manière non permanente à l'autre partie de mâchoire (156).

7. Pince selon une ou plusieurs des revendications 1 à 6, caractérisée en ce que l'adaptateur (60; 100; 110; 138; 162, 164) présente des moyens d'encliquetage (78, 80) qui peuvent s'encliqueter avec des moyens d'encliquetage (88, 90) correspondants d'une partie de mâchoire (24 ; 132 ; 154, 156) à laquelle l'adaptateur (60 ; 100 ; 110 ; 138 ; 162, 164) peut être fixé.

8. Pince selon une ou plusieurs des revendications 1 à 7, caractérisée en ce que l'adaptateur (60 ; 100 ; 110) présente un corps en U.

9. Pince selon la revendication 8, caractérisée en ce que deux branches (62, 64 ; 102, 104 ; 118, 120) du corps en U sont reliées entre elles par une entretoise (70), l'entretoise (70) passant sur la partie de mâchoire (24) à laquelle l'adaptateur (60 ; 100 ; 110) peut être fixé, lorsque l'adaptateur (60 ; 100 ; 110) est fixé.

10. Pince selon une ou plusieurs des revendications 1 à 9, caractérisée en ce que la partie de mâchoire (24) à laquelle l'adaptateur (60 ; 100 ; 110) peut être fixé présente une rainure (82) qui s'étend dans la direction longitudinale de la partie de mâchoire (24), qui est disposée périphériquement et dans laquelle s'engagent des saillies (76) correspondantes de l'adaptateur (60 ; 100 ; 110).

11. Pince selon une ou plusieurs des revendications 1 à 10, caractérisée en ce que l'adaptateur (60 ; 100 ; 110 ; 138 ; 162, 164) est réalisé d'un seul tenant en métal.
